(19) Europäisches Patentamt
European Patent Office
Office européen des brevets

(11) EP 1 294 366 B1

(12) **EUROPEAN PATENT SPECIFICATION**

(45) Date of publication and mention of the grant of the patent:
**18.08.2004 Bulletin 2004/34**

(21) Application number: **01934724.4**

(22) Date of filing: **08.05.2001**

(51) Int Cl.[7]: **A61K 31/00**, A61K 31/136, A61K 31/4706, A61P 11/00

(86) International application number:
**PCT/SE2001/001014**

(87) International publication number:
**WO 2001/085146 (15.11.2001 Gazette 2001/46)**

(54) **PHARMACEUTICAL COMPOUNDS FOR TREATING COPD**

PHARMAZEUTISCHE VERBINDUNGEN ZUR BEHANDLUNG VON CHRONISCH-OBSTRUKTIVER BRONCHOPNEUMONIE

COMPOSES PHARMACEUTIQUES POUR LE TRAITEMENT DE LA BRONCHO-PNEUMOPATHIE CHRONIQUE OBSTRUCTIVE

(84) Designated Contracting States:
**AT BE CH CY DE DK ES FI FR GB GR IE IT LI LU MC NL PT SE TR**
Designated Extension States:
**SI**

(30) Priority: **12.05.2000 GB 0011358**

(43) Date of publication of application:
**26.03.2003 Bulletin 2003/13**

(73) Proprietor: **AstraZeneca AB**
**151 85 Södertälje (SE)**

(72) Inventors:
- **Thong, Kam-Wah**
  **Loughborough, Leics. LE11 5RH (GB)**
- **KETTLE, Anthony, J. Free Radical Research group**
  **Christchurh (NZ)**

(56) References cited:
**WO-A1-00/51598**

- **W.J. MARTIN II ET AL.: 'Reduction of neutrophil-mediated injury to pulmonary endothelial cells by dapsone' AM. REV. RESPIR. DIS. vol. 131, 1985, pages 544 - 547, XP002945555**
- **ANTHONY J. KETTLE ET AL.: 'Mechanism of inhibition of myeloperoxidase by anti-inflammatory drugs' BIOCHEMICAL PHARMACOLOGY vol. 41, no. 10, 1991, pages 1485 - 1492, XP002945556**
- **S.W. CROOKS ET AL.: 'Bronchial inflammation in acute bacterial exacerbations of chronic bronchitis: the role of leukotriene B4' EUR. RESPIR. J. vol. 15, 2000, pages 274 - 280, XP002945557**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 294 366 B1

## Description

The present invention relates to the use of certain pharmaceutical compounds for the treatment of COPD.

COPD is a major cause of morbidity and mortality. A key etiological factor is smoking. It is apparent that smokers have elevated levels of MPO (Dash *et al*., Blood., 1991, 72, 1619; Bridges *et al*., Eur. J. Respir. Dis., 1985, 67, 84). Furthermore, there is circumstantial evidence to link MPO levels with the severity of lung disease in human subjects (Hill *et al*., Am. J. Respir. Crit. Care., 1999, 160, 893; Keatings & Barnes., Am. J. Respir. Crit. Care., 1997, 155, 449; Regelmann *et al*., Pediatric. Pulmonol., 1995, 19, 1; Linden *et al*., Am Rev. respir. Dis., 1993, 148, 1226). Myeloperoxidase is a heme protein that plays a vital role in the generation of toxic hypochlorus acid and free radicals, which may be involved in cellular damage and inflammation (Kettle & Winterbourn., Curr. Opin. Hematol., 2000, 7, 53). This protein has been implicated in a variety of different conditions (Klebanoff., Proc. Assoc. Am. Physicians., 1999, 111, 383). Compounds having activity as inhibitors of MPO are known in the art (Kettle & Winterbourn., Biochem. Pharmacol., 1991,41, 10; Bozeman *et al*., Biochem. Pharmacol., 1992, 44, 553). For example, the compound dapsone, which is known to be an inhibitor of MPO has been linked to the treatment of various conditions, including a general reference to inflammatory diseases such as asthma (Berlow *et al*., J. Allergy Clin. Immunol., 1991, 87, 710). Interestingly, there is no specific mention of any synthetically derived chemical inhibitors of MPO being use for the treatment of COPD.

Current drugs used for treating COPD are not all fully effective. The need for novel and better drugs is essential to cope with the rising incidence of COPD (Peleman *et al*., Curr. Opin. Cardiovas. Pulmonary. Renal. Invest. Drugs., 1999, 1, 491). It has now surprisingly been found that compounds having activity as inhibitors of MPO are expected to be of potential use in the treatment of COPD.

In a first aspect the invention therefore provides the use of an MPO inhibitor or a pharmaceutically acceptable salt thereof in the manufacture of a medicament for the prevention or treatment of COPD. It will be understood that the MPO inhibitors of the invention can be used therapeutically or as prophylactics.

Particularly suitable compounds include MPO inhibitors known in the art.

Preferred compounds include those listed below:

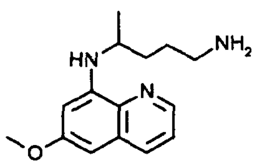

PRIMAQUINE

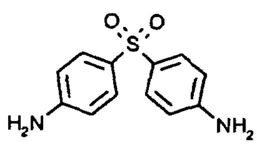

DAPSONE

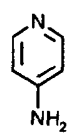

AMINOPYRINE

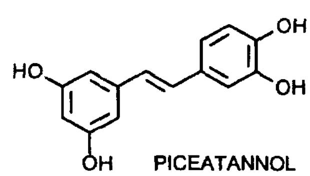

PICEATANNOL

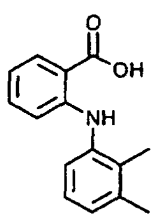

MEFENAMIC ACID

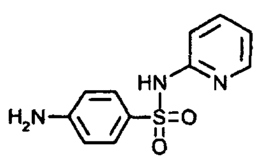

SULFAPYRIDINE

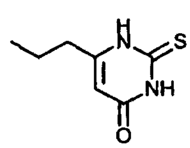

PROPYLTHIOURACIL

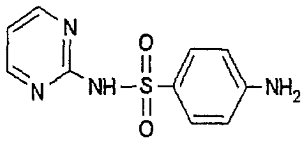

SULFADIAZINE

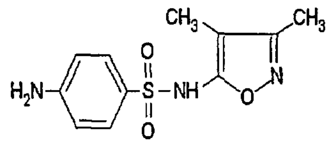

SULFISOXAZOLE

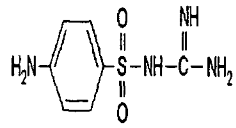

SULFAGUANIDINE

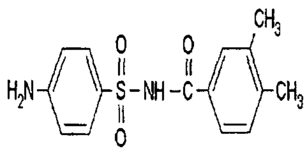

SULFANITRAN

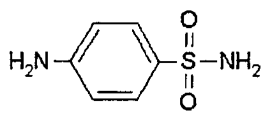

SULFANILAMIDE

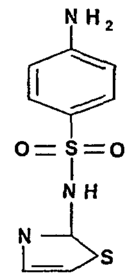

N-1(2 THIAZOLYL)SULFANILAMIDE

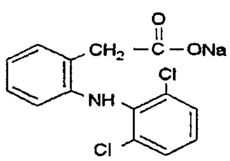

DICLOFENAC

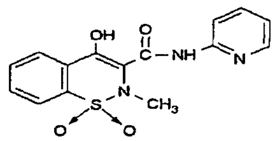

PIROXICAM

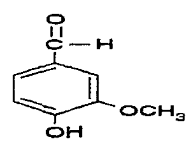

VANILLIN

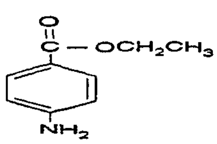

ETHYL AMINOBENZOATE

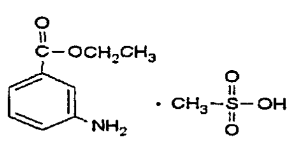

3 AMINO ACID ETHYLESTER

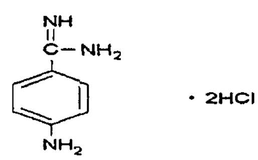

p-AMINOBENZAMIDINE

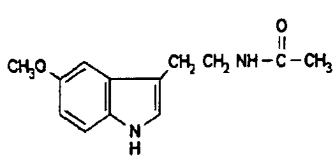

MELATONIN

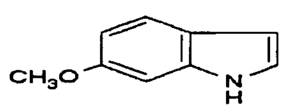

6 METHOXYINDOLE

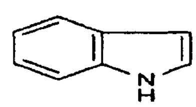

INDOLE

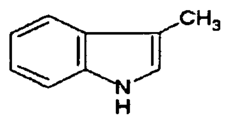

3-METHYLINDOLE

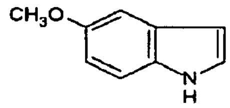

5-METHOXYINDOLE

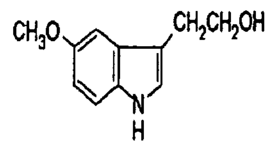

5 METHOXYTRYPTOPHOL

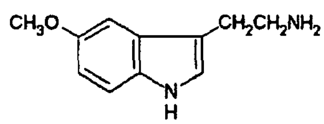

5 METHOXYTRYPTAMINE

Additional preferred compounds include the following:

ISONIAZID
NITECAPONE
5-AMINOSALICYLIC ACID
PHENYLHYDRAZINE
D-PENICILLAMINE
TIOPRONIN
RESORCINOL
QUERCETIN
RUTIN
QUINACRINE
BAKUCHIOL

The above compounds can be used both as free bases and pharmaceutically acceptable salts. Suitable salts include all known pharmaceutically acceptable salts such as acid addition salts such as hydrochloride and malate salts.

Preferred compounds include primaquine, sulfanilamide, dapsone and sulfapyridine, in particular dapsone.

Suitable daily dose ranges are from about 0.1 mg/kg to about 100 mg/kg. Unit doses may be administered conventionally once or more than once a day, for example, 2, 3 or 4 times a day, more usually 1 or 2 times a day. A typical dosing regime for dapsone or propylthiouracil would be oral once or twice a day at 100 mg or 300mg, respectively.

The pharmaceutical composition comprising the MPO inhibitor of the invention may conveniently be in the form of tablets, pills, capsules, syrups, powders or granules for oral administration; sterile parental or subcutaneous solutions, suspensions for parental administration of suppositories for rectal administration, all of which are well known in the art.

The following examples illustrate the invention.

**Example 1**

Here we describe an *in vitro* MPO assay that was developed to assess inhibition of enzyme activity. Essentially the MPO assay was designed to measure the production of hypochlorus acid (HOCl), which is the key physiological product generated by the enzyme *in vivo*. An outline of the assay reactions is given:

$$1.\ H_2O_2 + Cl^- \xrightarrow{MPO} HOCl + H_2O$$

2. $HOCl + RNH_2$ (taurine) $\rightarrow$ RNHCl (taurine chloramine) + $H_2O$

3. RNHCl + I- + $H_2O \rightarrow RNH_2$ + HOI

H+

4. HOI + TMB ------------------------------> TMB (oxdised) + I- + H2O

The reaction mixtures in 20mM phosphate buffer pH6.5 contained 2.5nM MPO (purified human enzyme from Planta), 100uM $H_2O_2$, 140mM NaCl, 10mM taurine, 20uM tyrosine and compound solvent, DMSO, at 1%. Compounds were preincubated with the MPO enzyme in buffer for about 15min prior to start of reaction with $H_2O_2$. The whole reaction was carried out at room temperatutre for 10min in a 96-well plate. The reaction was terminated by a stop/ developing reagent, which consist in their final concentration of Glacial acetic acid (400mM), KI (100uM) and TMB in dimethylformamide (10mM). All test concentrations were done in duplicated with at least two separate determinations n=2, unless otherwise stated. The inhibitory concentration for a compound is presentated as pIC50, which is -log IC50.

Various compounds have been tested against the human MPO. It can be seen that dapsone is the most potent inhibitor of the sulfones/sulfonamides tested. Indoles and other compounds are also effective in blocking the production of HOCl by human MPO. All data obtained for the sulfones/sulfonamides, indoles and miscellaneous are presented in Table 1, 2 and 3, respectively.

Table 1:

| Inhibtion of human MPO-HOCl production by sulfones/sulfonamides | |
|---|---|
| **Compound** | **pIC50** |
| Dapsone | 6.2 |
| N-1(2 thiazolyl)-sulfanilamide | 6.0 |
| Sulfanilamide | 6.0 |
| Sulfapyridine | 5.7 |
| Sulfaguanidine | 5.5 |
| Sulfisoxazole | 5.2 |
| Sulfadiazine | 5.2 |
| Sulfanitran | 5.1 |

Table 2:

| Inhibition of human MPO-HOCl production by indoles | |
|---|---|
| **Compound** | **pIC50** |
| 5-Methoxytryptophol | 6.3 |
| 5-Methoxytryptamine | 6.2 |
| Melatonin | 6.1 |
| 3-Methylindole | 5.9 |
| 6-Methoxyindole | 5.8 |
| Indole | 5.7 |
| 5-Methoxyindole | 5.6 |

Table 3:

| Inhibition of human MPO-HOCl production | |
|---|---|
| **Compound** | **pIC50** |
| Ethyl aminobenzoate | 6.2 |
| 3-Aminobenzoic acid ethylester | 6.2 |
| p-Aminobenzamidine | 5.6 |
| Piroxicam | 5.6 (n=1) |
| Diclofenac | 5.4 |
| Vanillin | 5.1 |

**Example 2**

Here we describe the use of a functional human neutrophil assay to determine the effects of MPO inhibitors on the production of HOCl. This assay detects the production of HOCl from stimulated (e.g. PMA, LPS, fMLP, zymozan) human neutrophils. Human neutrophils were purified from fresh heparinised blood by density centrifugation on Polymorphprep (Nycomed). These neutrophils were used immediately after purification. A standard reaction mixture contained the following: 2 X 106 neutrophils, 140mM NaCl, 5mM taurine, 0.5mM MgCl2, 1mM $CaCl_2$ and 1mg/ml glucose. Test compounds were made up in DMSO and added to cells, with a final DMSO concentration of 0.5%. Test compounds were given 15min preincubation at 37C with neutrophils prior to the addition of the PMA stimulant (1μg/ml). The assay was then allowed to progress for another 30min at 37C. At the end of the incubation, supernatants were collected by centrifugation and assayed for HOCl by using the stop/development reagent as above. All compounds were tested in duplicate with at least two separate determinations n=2 from two different donors.

The data for some of these inhibitors are shown in Table 4.

Table 4:

| Inhibition of HOCl production by stimulated human neutrophils | |
|---|---|
| **HOCl production by neutrophils** | **pIC50** |
| Primaquine | 4.9 |
| Sufanilamide | 4.8 |
| Dapsone | 4.7 |
| Sulfapyridine | 4.5 |

We have also shown that under the assay conditions and concentrations of inhibitors used, human neutrophils were not affected by cytotoxicity, as assessed by the release of lactate dehydrogenase from damaged neutrophils. Lactate dehydrogenase activity was measured as described by Boehringer Mannheim GmbH, Sandhofer Strabe 116, D-68305 Mannheim, Germany (Cytotoxicity Detection Kit-LDH- Cat No: 1 644 793).

**Example 3**

There are several animal models of COPD, which can be employed for the testing of MPO inhibitors. These models have been referred in the reviews of Snider (Chest., 1992, 101, 74S) and Shapiro (Am. J. Respir. Cell Mol. Biol., 2000, 22, 4). In our study, we prefer the LPS- and/or smoking-induced lung injury rodent model. Mice or rats can be be dosed (by any of the following routes: ip, po, iv, sc or aerosol) with MPO inhibitors prior to LPS and/or smoking challenge. After an appropriate set interval, the animals are sacrificed and assessed for lung injury (similar to the work reported by Faffe *et al.*, Eur.Respir. J., 2000, 15, 85; Suntres & Shek., Biochem. Pharmacol., 2000, 59, 1155; Vanhelden *et al.*, Exp. Lung. Res., 1997, 23, 297). MPO activity of the lung lavage fluids (BAL), lung tissues, neutrophils and whole blood are then measured. Blood samples can be analysed for inflammatory cells and cytokines (e.g. TNFα). Histology and biochemical markers (e.g. chlorinated protein, lactate dehydrogenase, alkaline phosphatase) for lung cellular damage can be assessed. The efficacies of the MPO inhibitors are measured against their abilities to reduce/prevent lung injury. It is expected these MPO inhibitors will be therapeutically or prohylactically effective in these models.

## Claims

1. Use of an MPO inhibitor or a pharmaceutically acceptable salt thereof. in the manufacture of a medicament for the prevention or treatment of COPD.

2. Use according to claim 1 where the compound having MPO inhibitory activity is primaquine, dapsone, aminopyrine, piceatannol, mefenamic acid, sulfapyridine, sulfanilamide, propylthiouracil, sulfadiazine, sulfisoxazole, sulfaguanidine, sulfanitran, sulfanilamide, N-1(2-thiazolyl)sulfanilamide, diclofenac, piroxicam, vanillin, ethyl aminobenzoate, 3-aminoacid ethylester, p-aminobenzamide, melatonin, 6-methoxyindole, indole, 3-methylindole, 5-methoxyindiole, 5-methoxytryptophol, 5-methoxytryptamine and pharmaceutically acceptable salts thereof.

3. Use according to claim 1 where the compound having MPO inhibitory activity is primaquine, sulfanilamide, dapsone and sulfapyridine and pharmaceutically acceptable salts thereof.

4. Use according to claim 1 where the compound having MPO inhibitory activity is dapsone or a pharmaceutically acceptable salt thereof.

## Patentansprüche

1. Verwendung eines MPO-Inhibitors oder eines pharmazeutisch unbedenklichen Salzes davon zur Herstellung eines Medikaments zur Prävention oder Behandlung von chronisch-obstruktiver Atemwegserkrankung.

2. Verwendung nach Anspruch 1, wobei es sich bei der Verbindung mit MPO-hemmender Wirkung um Primaquine, Dapson, Aminopyrin, Piceatannol, Mefenaminsäure, Sulfapyridin, Sulfanilamid, Propylthiouracil, Sulfadiazin, Sulfisoxazol, Sulfaguanidin, Sulfanitran, Sulfanilamid, N-1(2-Thiazolyl)sulfanilamid, Diclofenac, Piroxicam, Vanillin, 4-Aminobenzoesäureethylester, 3-Aminobenzoesäureethylester, p-Aminobenzamidin, Melatonin, 6-Methoxyindol, Indol, 3-Methylindol, 5-Methoxyindol, 5-Methoxytryptophol, 5-Methoxytryptamin und deren pharmazeutisch unbedenkliche Salze handelt.

3. Verwendung nach Anspruch 1, wobei es sich bei der Verbindung mit MPO-hemmender Wirkung um Primaquin, Sulfanilamid, Dapson und Sulfapyridin und deren pharmazeutisch unbedenkliche Salze handelt.

4. Verwendung nach Anspruch 1, wobei es sich bei der Verbindung mit MPO-hemmender Wirkung um Dapson oder ein pharmazeutisch unbedenkliches Salz davon handelt.

## Revendications

1. Utilisation d'un inhibiteur de la MPO ou d'un sel pharmaceutiquement acceptable de celui-ci pour la fabrication d'un médicament destiné à la prévention ou au traitement de la BPCO.

2. Utilisation selon la revendication 1, dans laquelle le composant présentant une activité inhibitrice de la MPO est la primaquine, la dapsone, l'aminopyrine, le piceatannol, l'acide méfénamique, la sulfapyridine, le sulfanilamide, le propylthiouracile, la sulfadiazine, le sulfisoxazole, la sulfaguanidine, le sulfanitran, le sulfanilamide, le N-1(2-thiazolyl)sulfanilamide, le diclofénac, le piroxicam, la vanilline, l'aminobenzoate d'éthyle, l'ester éthylique de 3-aminoacide, le p-aminobenzamide, la mélatonine, le 6-méthoxyindole, l'indole, le 3-méthylindole, le 5-méthoxyindole, le 5-méthoxytryptophol, la 5-méthoxytryptamine et leurs sels pharmaceutiquement acceptables.

3. Utilisation selon la revendication 1, dans laquelle le composé présentant une activité inhibitrice de la MPO est la primaquine, le sulfanilamide, la dapsone et la sulfapyridine et leurs sels pharmaceutiquement acceptables.

4. Utilisation selon la revendication 1, dans laquelle le composé présentant une activité inhibitrice de la MPO est la dapsone ou un sel pharmaceutiquement acceptable de celle-ci.